Europäisches Patentamt

European Patent Office

Office européen des brevets

(11) Publication number: **0 421 825 A1**

# EUROPEAN PATENT APPLICATION

(21) Application number: 90402285.2

(22) Date of filing: **10.08.90**

(51) Int. Cl.⁵: **A61K 31/165**, A61K 9/00,
A61K 47/10

The title of the invention has been amended (Guidelines for Examination in the EPO, A-III, 7.3).

(30) Priority: **11.08.89 ZA 896154**

(43) Date of publication of application:
**10.04.91 Bulletin 91/15**

(84) Designated Contracting States:
**CH DE ES FR GB IT LI**

(71) Applicant: **KADEM FARMASEUTIESE PRODUKTIE B.K.**
**103 City Centre, 107 Church Street**
**Potchefstroom, Transvaal(ZA)**

(72) Inventor: **Lötter, Antonie Phillipus**
**4, University Street**
**Potchefstroom, Transvaal(ZA)**

(74) Representative: **Le Guen, Gérard et al**
**CABINET LAVOIX 2, place d'Estienne d'Orves**
**F-75441 Paris Cédex 09(FR)**

(54) **Paracetamol solutions.**

(57) The invention relates to an orally administrable solution comprising an analgesic as active ingredient and a solvent therefor which solvent contains polyethylene glycol.

EP 0 421 825 A1

THIS invention relates to analgesic containing solutions. More particularly this invention relates to an orally administrable solution which is an alcohol-free water-based paracetamol syrup with a relatively high paracetamol concentration.

It is well known that ethanol or propylene glycol or mixtures thereof are used as carriers for paracetamol in the common paracetamol syrups. In such preparations paracetamol concentrations of up to 24 mg/cm$^3$ are conventionally made up. One reason for this generally accepted upper limit in conventional formulations is that higher concentrations of paracetamol would require the presence of unacceptably high concentrations of ethanol. Another reason is the bad taste of propylene glycol which would likewise be required to be present in higher concentrations. The taste of this solvent is difficult to mask.

A further disadvantage of using ethanol is that, for obvious reasons it is undesirable to involve the use thereof in medicines intended for children.

An object of this invention is therefore to provide an ethanol-free analgesic syrup and especially a paracetamol syrup with a low concentration of propylene glycol and which contains paracetamol at a concentration of higher than 24 mg/cm$^3$. Thus the abovementioned disadvantages of ethanol will be avoided and the problems with propylene glycol will be reduced to some extent because of its low concentration.

According to a first aspect of the invention there is provided an orally administrable solution comprising an analgesic as active ingredient and a solvent therefor, which solvent contains polyethylene glycol. The solvent may be an aqueous solution of polyethylene glycol and more preferably it comprises an aqueous solution of polyethylene glycol 6000. The solvent preferably also includes at least one of, but preferably both propylene glycol and glycerine. Glycerine also acts as a sweetener.

The analgesic preferably comprises paracetamol and may also comprise paracetamol in combination with one or more of other analgesics and/or other active ingredients. The other active ingredients preferably comprise one or more of antihistamines and/or cough suppressants and/or decongestants. The antihistamines may comprise promethazine, hydrochloride, dexchlorpheneramine maleate and diphenylhydramine hydrochloride. The cough suppressants may comprise codeinphosphate, dextromethorphan hydrobromide and pholcodein and the decongestants may comprise pseudo-ephedrine hydrochloride and other ephedrine derivatives.

The solution preferably includes one or more sweeteners which may be one or more of the group comprising sodium cyclamate, sodium saccharine and other known sweeteners. More preferably, the sweeteners comprise a combination of all three of sodium cyclamate, sodium saccharine and glycerine.

The solution may further, and preferably include one or more preservatives which may be at least one of but preferably both methylhydroxybenzoate and propylhydroxybenzoate.

In a further preference the solution may also include at least one flavouring agent which may be one or both of menthol and peppermint flavour. The peppermint flavour is preferably the commercially available product known by the product code H&R LR46275 supplied by ........ .

More preferable a combination of both menthol and peppermint flavour H&R LR46275 is used as a flavouring agent.

Also preferably the solution may include one or more colouring agents. The colouring agent is preferably green, more preferably tartrazine-free and most preferably a food colouring agent.

In the most preferred form of the invention the solution is ethanol-free.

The paracetamol solution may contain paracetamol at a concentration of more than 24 mg/cm$^3$.

According to a second aspect of the invention a mixture of propylene glycol and polyethylene glycol is used as a solvent for solutions containing paracetamol as an active ingredient.

According to a third aspect of the invention there is provided a process for preparing a paracetamol solution which may comprise the steps of -

[a] dissolving polyethylene glycol in water;

[b] optionally dissolving preservatives in propylene glycol or optionally using propylene glycol as such;

[c] mixing the solutions of steps [a] and [b] if step [b] is used otherwise using step [a] as such and optionally adding glycerine thereto;

[d] adding the analgesic to the solution of step [c], heating the solution and stirring the solution until the analgesic is dissolved;

[e] optionally dissolving sweeteners in water and adding this solution to the solution of step [d] which is then allowed to reach room temperature;

[f] flavouring and/or colouring agents may be optionally added to a hot or cold solution of any one of the above steps depending on the solubility and/or heat sensitivity of the agents to be added.

One embodiment of the invention and a method of preparation thereof will now be described in more detail by way of example only.

The following compounds and their quantities are used to make a paracetamol syrup with a paracetamol concentration of 50 mg/cm$^3$.

| EXAMPLE 1 | |
|---|---|
| 1. Paracetamol | 5,0 g |
| 2. Propylene glycol | 15,0 cm$^3$ |
| 3. Polyethylene glycol 6000 | 14,0 g |
| 4. Glycerine | 10,0 cm$^3$ |
| 5. Menthol | 0,005 g |
| 6. Methylhydroxybenzoate | 0,120 g |
| 7. Propylhydroxybenzoate | 0,03 g |
| 8. Sodium cyclamate | 1,75 g |
| 9. Sodium saccharine | 0,175 g |
| 10. Green colouring agent [without tartrazine] | 0,001 g |
| 11. Peppermint flavouring agent H&R LR46275 | 0,15 cm$^3$ |
| 12. De-ionised water | to 100,0 cm$^3$ |

The polyethylene glycol 6000 is dissolved in 20,0 cm$^3$ of de-ionised water to render a pre-mix identified as solution A .

The methylhydroxybenzoate and propylhydroxybenzoate are dissolved in the propylene glycol to render a pre-mix identified as solution B .

Solutions A and B are mixed and the glycerine is added thereto, this mixture is identified as solution C .

The paracetamol is added to solution C and by heating the solution mildly to not more than 80° C under continuous stirring the paracetamol is completely dissolved to render solution D .

The sodium cyclamate and sodium saccharine are dissolved in 10,0 cm$^3$ de-ionised water and after the addition of the menthol, the solution is added to solution D and allowed to cool down.

When the solution has reached room temperature the green colouring agent as well as the peppermint flavouring agent H&R LR46275 are added. After vigorous stirring, the solution is made up to final volume [100 cm$^3$] with de-ionised water.

Finally the solution is filtered and packed.

It will be appreciated that many variations in detail of the invention are possible without thereby departing from the spirit of the invention. One such departure is for example where the flavouring and/or especially the colouring agent is omitted, since some people may be allergic to especially the colouring agent.

**Claims**

1. An orally administrable solution comprising an analgesic as active ingredient and a solvent therefor which solvent contains polyethylene glycol.

2. The solution of claim 1 wherein the solvent is an aqueous solution of polyethylene glycol.

3. The solution of either claim 1 or 2 wherein the polyethylene glycol is polyethylene glycol 6000.

4. The solution of any one of claims 1 to 3 wherein the solvent also includes at least one of propylene glycol and glycerine.

5. The solution of any one of claims 1 to 4 wherein the analgesic comprises paracetamol.

6. The solution of claim 5 which also includes one or more other analgesics and/or other active ingredients.

7. The solution of claim 6 wherein the other active ingredient comprises one or more of an antihistamine and/or a cough suppressant and/or a decongestant.

8. The solution of any one of claims 1 to 7 which includes one or more sweeteners.

9. The solution of claim 8 wherein the sweetener includes glycerine.

10. The solution of claim 9 wherein the sweetener comprises a combination of all three of sodium cyclamate, sodium saccharine and glycerine.

11. The solution of any one of claims 1 to 10 which includes one or more preservatives.

12. The solution of claim 11 wherein the preservative is one or both of methylhydroxybenzoate and propylhydroxybenzoate.

13. The solution of any one of claims 1 to 12 which includes one or more flavouring agents.

14. The solution of claim 13 wherein the flavouring agent is one or both of menthol and peppermint flavour.

15. The solution of any one of claims 1 to 14 which includes one or more colouring agents.

16. The solution of any one of claims 1 to 15 which is ethanol-free.

17. The solution of any one of claims 1 to 16 containing paracetamol at a concentration of more than 24 mg/cm$^3$.

18. A process for preparing a solution comprising the steps of -

[a] dissolving polyethylene glycol in water;

[b] optionally dissolving preservatives in propylene glycol or optionally using propylene glycol as such;

[c] mixing the solutions of steps [a] and [b] if step [b] is used otherwise use step [a] as such and optionally adding glycerine thereto;

[d] adding the analgesic to the solution of step [c], heating the solution and stirring the solution until the analgesic is dissolved;

[e] optionally dissolving sweeteners in water and adding the solution to the solution of step [d] which is then allowed to reach room temperature;

[f] optionally flavouring and/or colouring agents may be added to a hot or cold solution of any one of the above steps depending on the solubility and/or heat sensitivity of the agents to be added.

European
Patent Office

EUROPEAN SEARCH
REPORT

Application Number

EP 90 40 2285

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 590 212  (J.E. BERNSTEIN)<br>* Claims; column 1, lines 55-63; column 2, example 2 *<br>– – – | 1-2,4-5 | A 61 K<br>31/165<br>A 61 K 9/00<br>A 61 K 47/10 |
| A | WO-A-8 503 439  (SCHERER)<br>* Claims 1,6,28,33; page 7, lines 3-6; page 8, lines 23-26; page 9, example 1 *<br>– – – | 1-5 | |
| A | US-A-4 307 073  (E.B. NELSON)<br>* Claim 12; column 1, lines 53-55; column 2, lines 17-21,58-68; column 3, lines 4-6 *<br>– – – | 1-2,4-6, 13,15 | |
| A | WO-A-8 809 656  (A. SUNSHINE)<br>* Claims 1,19-20,23,30; page 24, example 2 *<br>– – – | 1-2,4-7, 13 | |
| P,X | EP-A-0 379 147  (STERLING DRUG)<br>* Claims 1-5; page 3, lines 47-53; page 4, lines 19-25 *<br>– – – – – | 1-16,18 | |

TECHNICAL FIELDS
SEARCHED (Int. Cl.5)

A 61 K

The present search report has been drawn up for all claims

| Place of search | Date of completion of search | Examiner |
|---|---|---|
| The Hague | 16 November 90 | SCARPONI U. |

CATEGORY OF CITED DOCUMENTS
X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons
......................................................................
& : member of the same patent family, corresponding document